# EUROPEAN PATENT SPECIFICATION

(11) **EP 4 297 710 B1**
(45) Date of publication and mention of the grant of the patent: **02.12.2026**
(21) Application number: 22718527.9
(22) Date of filing: 05.04.2022
(51) Int. Cl.: A61F 13/36, A61F 13/00, A61F 13/01

(54) **EXPANDABLE TAMPONADE**
HÄMOSTASEEINHEIT UND ANORDNUNG DAMIT
UNITÉ POUR L'HÉMOSTASE ET DISPOSITIF LE CONTENANT

(30) Priority: 05.04.2021 US 202163170700 P
(43) Date of publication of application: 03.01.2024
(73) Proprietor: Biolife, LLC, Sarasota, FL 34243 (US)
(72) Inventor: KEENE, Talmadge, Kelly, Ruskin, FL 33570 (US); FORDHAM, John, Tifton, Indialantic, FL 32903 (US)
(74) Representative: Bradley, Adrian
(86) International application number: PCT/US2022/023509
(87) International publication number: WO 2022/216726

(56) References cited:
- WO-A1-2010/129587
- US-A1- 2008 139 988
- US-A1- 2014 142 523
- US-A1- 2019 209 389

## Description

### RELATED APPLICATION

### FIELD OF THE INVENTION

The present invention relates generally to devices for diminishing or stopping the bleeding of a wound, particularly a deep wound.

### BACKGROUND OF THE INVENTION

US2008/139988 A1 describes a bandage including a strip of fabric for winding about a body portion having a wound, a dressing, formed integrally with said strip of fabric, for placement over the wound, and a pocket formed over said dressing, adapted to contain therein a topical agent for treating the wound. US 2014/142523 A1 describes a self-expanding wound dressing that includes a first outer layer, a second outer layer, and a liquid-expandable layer disposed between the first outer layer and the second outer layer, wherein the liquid-expandable layer includes a plurality of liquid-expandable articles retained by a substrate. US 2019/209389 A1 describes a unit configured to be directly applied to a bleeding wound and comprising an envelope enclosing an inner space as well as an effective amount of a hemostatic material disposed within the inner space. WO 2010/129587 A1 describes a composition comprising a plurality of small, liquid-expandable articles and their use to induce hemostasis when applied to bleeding wounds. Bleeding from certain wounds, such as deep wounds, cannot be controlled by simple topical hemostasis devices and methods. Oftentimes, such simple devices and methods are not sufficient to control the bleeding beneath the skin of the subject who has incurred a deep wound. Examples of such deep wounds include gun shots, knife stabbings, or other puncture-type wounds. Other examples include ulcerative-type wounds. These wounds often damage blood vessels within the body and those vessels require more localized compression that can be accomplished by topical compression.

The Food & Drug Administration recently created a clearance path for packing products that are used to temporarily control internal bleeding, as long as the products are not biosorbable. These products typically comprise a long narrow sheet of gauze, folded accordion style. To stop bleeding, this material is packed over and over again into the wound to control bleeding. Some of the material is left outside the wound so that the product can be pulled from the wound once the patient is in a hospital setting where internal bleeding can be treated via surgical means.

For a bullet wound, the entry wound may be quite small, but the track of the projectile may create a much larger wound channel beneath the skin. It may take a substantial amount of wound packing gauze to fill the wound channel. This material cannot completely expand into a massive ball as such expansion would make it very difficult to remove the gauze from the skin entry wound.

Super absorbing polymers (SAP) have been used in diapers and other products since the late 1960's. SAP was used in tampons until the 1980's, but it was discontinued due to toxic shock syndrome. Although use of SAP was discontinued, the syndrome only occurred if the tampon was unchanged for an extended period of time or was forgotten and left inside the person. Highly absorbent cotton polymer or rayon polymer tampons are used today.

Absorbing bandages and honey were used in ancient times to control bleeding at wound sites. Since the 1960, absorbing polymers and hydrogels have been used in surgery and wound care with the first reporting of the use of hydrogels being made by Wichterle & Lim.

### SUMMARY OF THE INVENTION

Briefly, the present invention is directed to an expandable device, called a tamponade, that can be used for hemostasis control of wounds. The invention is in accordance with appended claim 1. Various optional features are as set out in the appended dependent claims.

In one embodiment, at least two individual pouches, each containing an expandable material enclosed within a blood-permeable material, are connected in a series and are designed to be inserted into a wound wherein the individual pouches expand upon contact with blood or other bodily fluid to create compression and curtail bleeding from the wound.

In certain embodiments, the expandable material is an absorbent material.

In other embodiments, the individual pouches, including either the expandable material and the blood-permeable material, or both, may be formed from materials that have hemostatic properties. In one such example, the exterior liquid-permeable material includes a hemostatic coating thereon.

There may be provided a composition useful as a wound sealing powder that comprises a particulate powder consisting essentially of a substantially anhydrous salt ferrate compound combined with an effective amount of an insoluble cation exchange material wherein the particle size distribution range of the particles in the powder is 160 microns or less.

In another embodiment, there is provided a wound sealing composition comprising a particulate powder consisting essentially of a substantially anhydrous salt ferrate compound combined with an effective amount of an insoluble cation exchange material wherein the majority of the particles in the powder have particle sizes of less than approximately 48 microns.

In another embodiment, there is provided a wound sealing composition comprising a particulate powder consisting essentially of a substantially anhydrous salt ferrate compound combined with an effective amount of an insoluble cation exchange material wherein the powder contains essentially no particles having a particle size of 158 microns or more.

There is described herein a method of making a particulate powder for a wound sealing composition wherein the powder consists essentially of a substantially anhydrous salt ferrate compound combined with an effective amount of an insoluble cation exchange material wherein the powder contains essentially no particles having a particle size of 158 microns or more, the method comprising steps of drying an insoluble cation exchange material to a moisture content of approximately 3% or less; mixing a substantially anhydrous salt ferrate compound having an average particle size of 2 mm or less with the cation exchange material at a weight ratio of approximately 1 to 2, ferrate to cation exchange material; providing the dried cation exchange material at an average particle size of less than about 70; blending the mixed 1:2 ferrate:cation exchange material with the dried cation exchange material having an average particle size of less than about 70 microns to obtain an approximate 1 to 7 weight mixture of ferrate to cation exchange material.

There is described herein a method of arresting or reducing the blood flow from a wound on a patient having a blood-letting wound comprising the steps of applying a wound sealing composition comprising a particulate powder consisting essentially of a substantially anhydrous salt ferrate compound combined with an effective amount of an insoluble cation exchange material wherein the majority of the particles in the powder have particle sizes of less than approximately 48 microns and allowing a seal to form over the wound so that blood flow from the wound is reduced.

In another embodiment, there is provided a wound sealing composition comprising a particulate powder consisting essentially of a ground insoluble cation exchange material wherein the particle size distribution range of the particles in the powder is 160 microns or less. And, in another embodiment, there is provided a wound sealing composition comprising a particulate powder consisting essentially of a ground insoluble cation exchange material wherein the majority of the particles in the powder have particle sizes of less than approximately 48 microns.

In yet another embodiment, there is provided a wound sealing composition comprising a particulate powder consisting essentially of an insoluble cation exchange material wherein the particle size distribution range of the particles in the powder is 160 microns or less and the moisture content of the powder is 20% or less. And, in yet another embodiment, there is provided a particulate powder consisting essentially of a ground insoluble cation exchange material wherein the majority of the particles in the powder have particle sizes of less than approximately 48 microns and the moisture content of the powder is 20% or less.

### BRIEF DESCRIPTION OF THE DRAWINGS

Figure 1 is a cross-section side view of an individual pouch useable according to the present invention and prior to the pouch being inserted into a wound and expanding.
Figure 2 is a cross-section side view showing the pouch of Figure 1 prior to the pouch being inserted into a wound and expanding and transitioned into an expanded pouch after the pouch is wetted, such as after insertion into a wound and after expanding.
Figure 3 is cross-section top view of a series of pouches wherein individual pouches are connected via a connecting device.
Figure 4 is cross-section side view of a series of pouches wherein individual pouches are connected via a tube from which the pouches have been formed with the tube portions being sealed between individual pouches.
Figure 5 is a cross-section side view of what is shown in Figure 4 being inserted into a wound.
Figure 6 is a side view of an embodiment of a series of individual pouches of the present invention.

### DETAILED DESCRIPTION OF PREFERRED EMBODIMENTS

Reference now will be made in detail to the embodiments of the invention, one or more examples of which are set forth below. Each example is provided by way of explanation of the invention, not a limitation of the invention. For instance, features illustrated or described as part of one embodiment, can be used on another embodiment to yield a still further embodiment. The following embodiments and aspects thereof are described and illustrated in conjunction with systems, tools and methods which are meant to be exemplary and illustrative and not limiting in scope. In various embodiments one or more of the above-described problems have been reduced or eliminated while other embodiments are directed to other improvements.

Thus, it is intended that the present invention covers such modifications and variations as come within the scope of the appended claims and their equivalents. Other objects, features and aspects of the present invention are disclosed in or are obvious from the following detailed description. It is to be understood by one of ordinary skill in the art that the present discussion is a description of exemplary embodiments only, and is not intended as limiting the broader aspects of the present invention.

It should be understood that the examples and embodiments described herein are for illustrative purposes only and that various modifications or changes in light thereof will be suggested to persons skilled in the art and are to be included within the spirit and purview of this application and the scope of the appended claims. In addition, any elements or limitations of any invention or embodiment thereof disclosed herein can be combined with any and/or all other elements or limitations (individually or in any combination) or any other invention or embodiment thereof disclosed herein, and all such combinations are contemplated with the scope of the invention without limitation thereto.

The pouches of the present invention are connected in such a way that when one pouch is removed from the body, it pulls on the next pouch and so on, allowing all of the pouches to be removed without damaging the wound walls. The pouches are designed to simulate the size of the wound in which they are used. The individual pouches will prevent the swelling, due to the expanding polymer, from creating mass with a diameter that would damage the wound as it is removed.

The expanding polymer may be a powder, fibrous material, or a combination of both. The exterior of the pouch or pouch-forming material could be a blood permeable material or a mesh that contains the expanding polymer and is strong enough to prevent tearing during expansion and/or removal. The exterior pouch material may incorporate or be coated with a hemostatic material or a material that interferes with the wetting rate of the expanding polymer.

The pouches may be square, round, oblong, or any other shape that is suitable for insertion into a wound. The pouches may be all the same shape or the leading pouch may be designed to be smaller or a more conical shape to allow for easier insertion into the wound.

The pouches may or may not be radiopaque. The purpose for radiopaque is to allow hospitals to electronically ensure that all of the material is removed from the wound prior to closing the wound surgically.

Examples of hemostat coatings for the pouches include, but are not limited to: Multi-Valent cation salts (Iron, Aluminum, Calcium); Thrombin; Fibrinogen; Chitosan; Calcium alginate; Kaolin; Activate cellulose; Polyvinyl alcohol (PVA); Polylactic acid; Vit C, collagen, zinc or other wound healing materials.

Examples of wetting retardation materials include, but are not limited to: Calcium alginate; Sugars; Polysaccharides; PVA; Often a very dry material can have hydrophobic properties until initial wetting; Hydrogels; Hydrocolloids; Starches; and Other oils or lubricants.

The pouches can be formed from various materials known in the art such as Cotton, Polyethylene, Polypropylene, Rayon, Teflon, Spandex, and Silk.

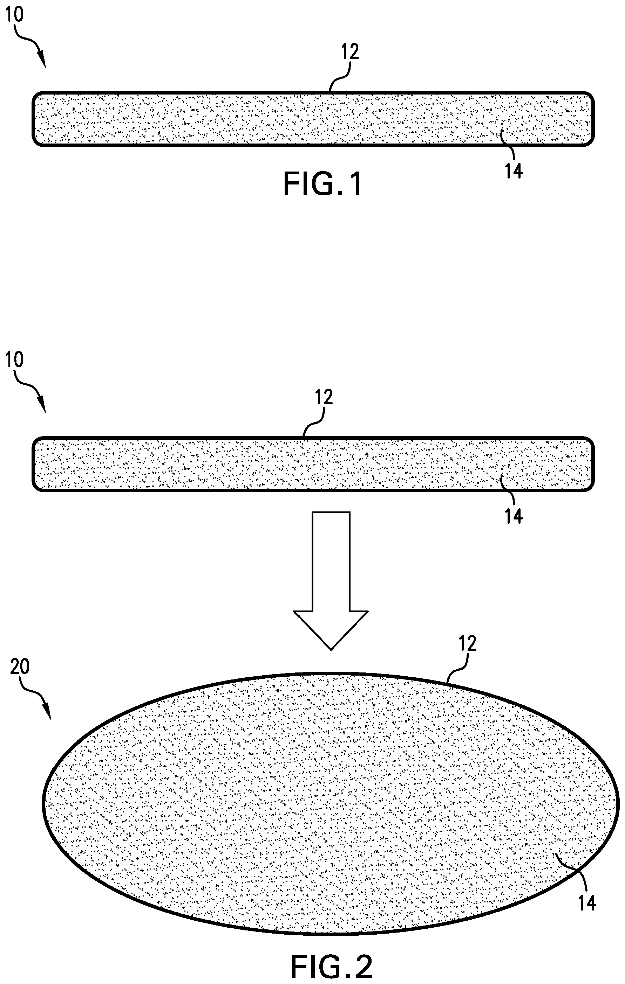

The expanding polymer materials include, but are not limited to: Any low cross-linked absorbent polymer such as Polyacrylates, Polysaccharides, and Ion exchange resins; Rayon; Cotton; and/or Cellulose. A cross-section top view of a pouch 10 according to the present invention, before insertion into the wound and expansion, is shown in Figure 1. As shown therein, an expandable material 14, such as a polymer powder, fibrous material, or combination thereof, is enclosed within pouch 10 formed from an exterior material 12, which may comprise a mesh or other blood-permeable material.

Figure 2 illustrates the expansion of the expandable material 14 within pouch 10 upon being wetted by blood or other bodily fluid after insertion into a wound. Pouch 20 has expanded to increase the area of the expandable material 14 housed within the exterior material 12 so that the space inside of a wound is at least partially filled so as to create compression inside the wound to stop bleeding.

As stated above, pouches 10 may be of different sizes, shapes. In addition, the number of individual pouches 10 connected together may vary for different usages.

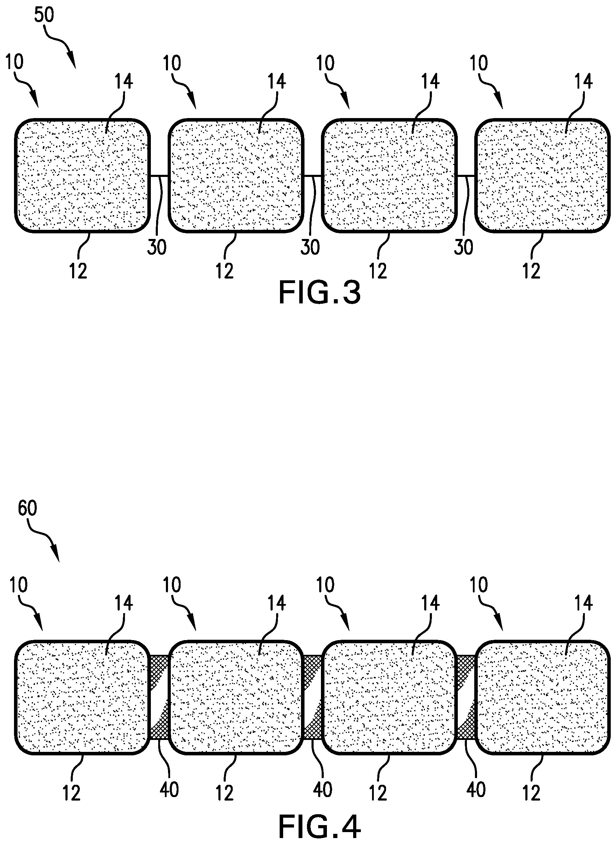

As shown in the top views in Figure 3, a series 50 of pouches 10 filled with expandable material 14 enclosed within an exterior material 12 are connected. The connections may be by any means to create a series of connected pouches 10, with one example being the use of strings 30 between each individual pouch 10 to connect the pouches together in a series 50.

Another means for connecting individual pouches 10 together in a series 60 is shown in the top views of Figure 4. In this embodiment of the present invention, a series 60 of individual pouches 10 is created by placing expandable material 14 into a tube formed of a sealable material (such as a plastic) and then sealing the tube portions 40. The resulting series 60 of individual pouches 10 are connected and are able to be used for larger wounds that required more than just an individual pouch. The number of individual pouches 10 connected together in the series 50 or the series 60 is variable, depending on the particular need.

The pouches may be coated with or have incorporated therein, a control agent, or otherwise be designed, to slow the wetting of the expanding polymer. This would allow the wound to be properly packed before the polymer begins to expand. These retardants may also include an oil or other lubricant to help the device slide down the wound tract.

The devices also may incorporate an antimicrobial agent.

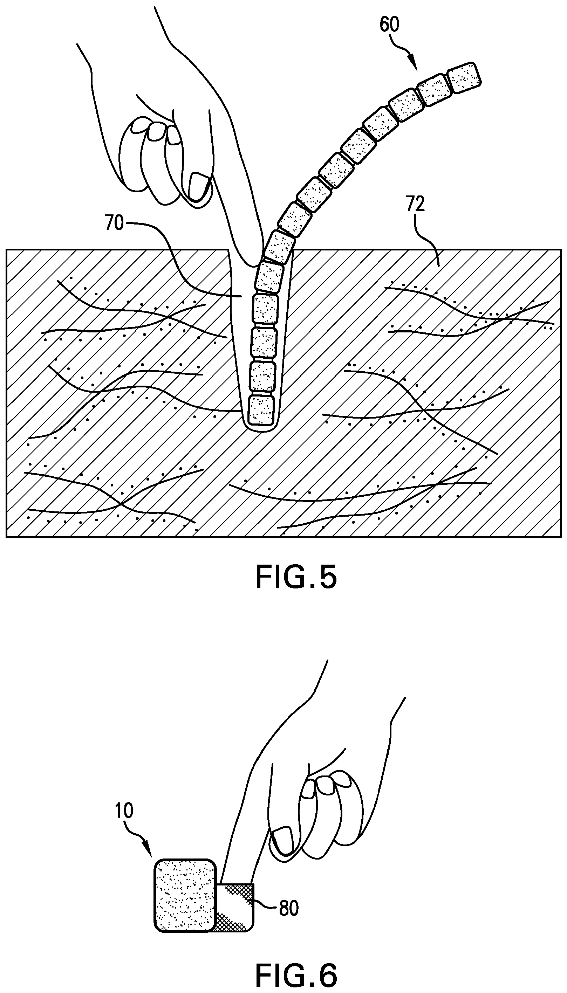

Figure 5 illustrates the series 60 of expandable individual pouches 10 shown in Figure 4 being inserted into a wound 70 in human tissue 72.

As shown in side view in Figure 6, an individual pouch 10 includes a pocket 80, constructed of any material, including the mesh or blood-permeable material, to assist insertion of the expandable pouch 10 into the wound. One particular embodiment, as shown in Figure 6, allows one's finger to be inserted into pocket 80 to push the pouch 10 into a wound. The pocket 80, or side-pouch, may be designed to fit a human finger or a swab or other utensil to insert the pouches. Pocket 80 may be included on all pouches in a series of individual pouches 10, or on only the first individual pouch 10 of the series.

## Claims

1. An expandable tamponade for hemostasis control of wounds comprising:
at least two sealed pouches (10) connected together, wherein each of the at least two sealed pouches (10) comprises an expandable material (14) enclosed within a liquid-permeable exterior material (12) and wherein the expandable material (14) expands upon being wetted with a liquid;
wherein at least one of the pouches (10) comprises a pocket (80) to assist in inserting the tamponade into the wound.

2. The expandable tamponade of claim 1 wherein the liquid-permeable exterior material (12) is blood permeable and the expandable material (14) is capable of expanding upon being wetted with blood.

3. The expandable tamponade of claim 1 wherein the expandable material (14) is an absorbent material.

4. The expandable tamponade of claim 1 wherein the at least two sealed pouches (10) are formed from one or more materials that have hemostatic properties.

5. The expandable tamponade of claim 1 wherein the at least two sealed pouches (10) comprise a hemostatic coating on the liquid-permeable exterior material (12).

6. The expandable tamponade of claim 1 wherein the at least two sealed pouches (10) are formed from a material that allows for blood transfer to the expandable material (14) within the pouch (10).

7. The expandable tamponade of claim 1 comprising at least three sealed pouches (10) connected together.

8. The expandable tamponade of claim 1 comprising at least four sealed pouches (10) connected together.

9. The expandable tamponade of claim 1 wherein the at least two sealed pouches (10) comprise an antimicrobial agent either on the liquid-permeable exterior or within the pouches (10).

10. The expandable tamponade of claim 1 wherein the pocket (80) is configured to fit a human finger, swab, or other insertion utensil.

11. The expandable tamponade of claim 1 wherein more than one of the pouches (10) comprises the pocket (80).

12. The expandable tamponade of claim 11 wherein all of the pouches (10) comprise the pocket (80).

## Patentansprüche

1. Expandierbare Tamponade zur Hämostasekontrolle von Wunden, die Folgendes umfasst:
mindestens zwei abgedichtete Beutel (10), die miteinander verbunden sind, wobei jeder der mindestens zwei abgedichteten Beutel (10) ein expandierbares Material (14) umfasst, das innerhalb eines flüssigkeitsdurchlässigen äußeren Materials (12) eingeschlossen ist, und wobei das expandierbare Material (14) bei Benetzung mit einer Flüssigkeit expandiert;
wobei mindestens einer der Beutel (10) eine Tasche (80) umfasst, um die Einführung der Tamponade in die Wunde zu unterstützen.

2. Expandierbare Tamponade nach Anspruch 1, wobei das flüssigkeitsdurchlässige äußere Material (12) für Blut durchlässig ist und das expandierbare Material (14) bei Benetzung mit Blut expandieren kann.

3. Expandierbare Tamponade nach Anspruch 1, wobei das expandierbare Material (14) ein saugfähiges Material ist.

4. Expandierbare Tamponade nach Anspruch 1, wobei die mindestens zwei abgedichteten Beutel (10) aus einem oder mehreren Materialien ausgebildet sind, die hämostatische Eigenschaften aufweisen.

5. Expandierbare Tamponade nach Anspruch 1, wobei die mindestens zwei abgedichteten Beutel (10) eine hämostatische Beschichtung auf dem flüssigkeitsdurchlässigen äußeren Material (12) umfassen.

6. Expandierbare Tamponade nach Anspruch 1, wobei die mindestens zwei abgedichteten Beutel (10) aus einem Material ausgebildet sind, das eine Blutübertragung zu dem expandierbaren Material (14) innerhalb des Beutels (10) ermöglicht.

7. Expandierbare Tamponade nach Anspruch 1, die mindestens drei abgedichtete Beutel (10) umfasst, die miteinander verbunden sind.

8. Expandierbare Tamponade nach Anspruch 1, die mindestens vier abgedichtete Beutel (10) umfasst, die miteinander verbunden sind.

9. Expandierbare Tamponade nach Anspruch 1, wobei die mindestens zwei abgedichteten Beutel (10) ein antimikrobielles Mittel entweder auf dem flüssigkeitsdurchlässigen Äußeren oder innerhalb der Beutel (10) umfassen.

10. Expandierbare Tamponade nach Anspruch 1, wobei die Tasche (80) zur Einpassung eines menschlichen Fingers, Tupfers oder anderen Einführungsinstruments konfiguriert ist.

11. Expandierbare Tamponade nach Anspruch 1, wobei mehr als einer der Beutel (10) die Tasche (80) umfasst.

12. Expandierbare Tamponade nach Anspruch 11, wobei alle der Beutel (10) die Tasche (80) umfassen.

## Revendications

1. Tamponnement expansible destiné au contrôle hémostatique des plaies, comprenant :
au moins deux pochettes scellées (10) reliées entre elles, où chacune parmi les au moins deux pochettes scellées (10) comprend un matériau expansible (14) renfermé au sein d'un matériau extérieur perméable aux liquides (12), et où le matériau expansible (14) se dilate lorsqu'il est imprégné par un liquide ;
dans lequel au moins l'une des pochettes (10) comprend une poche (80) destinée à faciliter l'insertion du tamponnement dans la plaie.

2. Tamponnement expansible selon la revendication 1, dans lequel le matériau extérieur perméable aux liquides (12) est perméable au sang et le matériau expansible (14) est capable de se dilater lorsqu'il est imprégné par du sang.

3. Tamponnement expansible selon la revendication 1, dans lequel le matériau expansible (14) est un matériau absorbant.

4. Tamponnement expansible selon la revendication 1, dans lequel les au moins deux pochettes scellées (10) sont formées d'un ou plusieurs matériaux présentant des propriétés hémostatiques.

5. Tamponnement expansible selon la revendication 1, dans lequel les au moins deux pochettes scellées (10) comprennent un revêtement hémostatique sur le matériau extérieur perméable aux liquides (12).

6. Tamponnement expansible selon la revendication 1, dans lequel les au moins deux pochettes scellées (10) sont formées d'un matériau permettant le transfert de sang vers le matériau expansible (14) à l'intérieur de la pochette (10).

7. Tamponnement expansible selon la revendication 1, comprenant au moins trois pochettes scellées (10) reliées entre elles.

8. Tamponnement expansible selon la revendication 1, comprenant au moins quatre pochettes scellées (10) reliées entre elles.

9. Tamponnement expansible selon la revendication 1, dans lequel les au moins deux pochettes scellées (10) comprennent un agent antimicrobien soit sur la surface extérieure perméable aux liquides, soit au sein des pochettes (10).

10. Tamponnement expansible selon la revendication 1, dans lequel la poche (80) est configurée pour s'adapter à un doigt humain, à un écouvillon ou à tout autre instrument d'insertion.

11. Tamponnement expansible selon la revendication 1, dans lequel plus d'une parmi les pochettes (10) comprennent la poche (80).

12. Tamponnement expansible selon la revendication 11, dans lequel toutes les pochettes (10) comprennent la poche (80).
